# EUROPÄISCHE PATENTANMELDUNG

(11) **EP 4 659 852 A1**
(43) Veröffentlichungstag der Anmeldung: **10.12.2025**
(21) Anmeldenummer: 24179486.6
(22) Anmeldetag: 03.06.2024
(51) Int. Cl.: B01F 27/213, B01F 27/231, B01F 33/501, B01F 35/32, B01F 101/20

(54) **ADAPTER FÜR EIN MISCHSYSTEM ZUM BEREITSTELLEN EINES KNOCHENZEMENTTEIGS UND KIT ZUM BEREITSTELLEN EINES KNOCHENZEMENTTEIGS UMFASSEND EINEN ADAPTER UND EIN MISCHSYSTEM**

(71) Anmelder: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Heraeus IP

(57) **Zusammenfassung**

Die Erfindung betrifft einen Adapter (100) für ein Mischsystem (200) zum Bereitstellen eines Knochenzementteigs aus zwei Ausgangskomponenten, umfassend
ein Befestigungselement (110) zur reversiblen Befestigung des Adapters an einem Handgriff (210) des Mischsystems, wobei der Handgriff eingerichtet ist, die zwei Ausgangskomponenten in einem Behälter (220) des Mischsystems mittels eines Mischstab (230) des Mischsystems zu mischen und so den Knochenzementteig bereitzustellen, und
ein Verbindungselement (120) zum reversiblen Verbinden des Adapters mit einer elektrischen Antriebsvorrichtung (400),
wobei der Adapter eingerichtet ist, eine kraftübertragende Verbindung zwischen der elektrischen Antriebsvorrichtung und dem Handgriff herzustellen, so dass der Mischstab maschinell mit der elektrischen Antriebsvorrichtung betreibbar ist.

Die Erfindung betrifft weiterhin ein Kit zum Bereitstellen eines Knochenzementteigs aus zwei Ausgangskomponenten umfassend einen derartigen Adapter und ein Mischsystem sowie ein Verfahren zum Bereitstellen eines Knochenzementteigs aus zwei Ausgangskomponenten mittels eines derartigen Kits.

## Beschreibung

Die Erfindung betrifft einen Adapter für ein Mischsystem zum Bereitstellen eines Knochenzementteigs aus zwei Ausgangskomponenten, umfassend
ein Befestigungselement zur reversiblen Befestigung des Adapters an einem Handgriff des Mischsystems, wobei der Handgriff eingerichtet ist, die zwei Ausgangskomponenten in einem Behälter des Mischsystems mittels eines Mischstab des Mischsystems zu mischen und so den Knochenzementteig bereitzustellen, und
ein Verbindungselement zum reversiblen Verbinden des Adapters mit einer elektrischen Antriebsvorrichtung,
wobei der Adapter eingerichtet ist, eine kraftübertragende Verbindung zwischen der elektrischen Antriebsvorrichtung und dem Handgriff herzustellen, so dass der Mischstab maschinell mit der elektrischen Antriebsvorrichtung betreibbar ist.

Die Erfindung betrifft weiterhin ein Kit zum Bereitstellen eines Knochenzementteigs aus zwei Ausgangskomponenten umfassend einen derartigen Adapter und ein Mischsystem sowie ein Verfahren zum Bereitstellen eines Knochenzementteigs aus zwei Ausgangskomponenten mittels eines derartigen Kits.

### Hintergrund der Erfindung

Es werden erhebliche Bemühungen unternommen, Vorrichtungen und Verfahren zum Bereitstellen von Knochenzement aufzuzeigen, mittels derer Knochenzementteig einfach, zuverlässig und schnell bereitgestellt werden kann. Ein wichtiger Aspekt bei der Bereitstellung von Knochenzementteig ist die Vermeidung von Lufteinschlüssen, wie beispielsweise Gasblasen, im Knochenzement. Zu deren Vermeidung wurden eine Vielzahl von Vakuum-Zementiersystemen beschreiben, von denen exemplarisch folgende genannt sind: US 6,033,105 A, US 5,624,184 A, US 4,671,263 A, US 4,973,168 A, US 5,100,241 A, WO 99/67015 A1, EP 1020167 A2, US 5,586,821 A, EP 1016452 A2, DE 3640279 A1, WO 94/26403 A1, EP 1005901 A2, EP 1886647 A1, US 5,344,232 A.

Es besteht im Markt der Wunsch zur Vereinfachung der Bereitstellung von Knochenzementteig. Eine Weiterentwicklung besteht in der Entwicklung von Mischsystemen, in denen beide Ausgangskomponenten in separaten Bereichen der Mischsysteme gelagert sind und erst unmittelbar vor der Zementierapplikation im Mischsystem selbst miteinander vermischt werden. Solche geschlossenen, sogenannten Full-Prepacked-Mischsysteme, sind beispielsweise in folgenden Schriften genannt: EP 0 692 229 A1, DE 10 2009 031 178 B3, US 5,997,544 A, US 6,709,149 B1, DE 698 12 726 T2, EP 0 796 653 A2, US 5,588,745 A.

In den vorgenannten Mischsystemen erfolgt die Vermischung einer Monomerflüssigkeit mit einem Knochenzementpulver durch mechanisches Vermischen, beispielsweise mittels eines handbetreibbaren Mischstabes.

Das Vorhandensein eines in das Mischsystem integrierten, handbetreibbaren Mischstabes hat für den Anwender den Vorteil, dass der Knochenzementteig ohne zusätzliche Hilfsmittel, wie beispielsweise einen Spatel, angemischt und bereitstellbar ist. Zudem minimiert ein in das Mischsystem integrierter Mischstab Anwendungsfehler durch den Anwender und erlaubt ein Bereitstellen des Knochenzementteigs unter möglichst sterilen Bedingungen.

Allerdings kann ein handbetriebener Mischstab, je nach Konsistenz und/oder Menge des finalen gewünschten Knochenzementteig, erhebliche Kraftanstrengungen beim Anmischen der Ausgangskomponenten nach sich ziehen. Eine maschinelle, insbesondere elektrische, Unterstützung bei der Bedienung des Mischstabs könnte das Anmischen der Ausgangskomponenten für den Anwender erleichtern.

Es besteht daher im Markt der Wunsch nach Systemen, welche möglichst einfach, anwendungssicher und gleichzeitig flexibel entweder per Handbetrieb oder durch maschinelle, insbesondere elektrische, Unterstützung, je nach Vorliebe des Anwenders des Mischsystems, zum Bereitstellen eines Knochenzementteigs geeignet sind und/oder umrüstbar sind.

### Aufgaben

Eine Aufgabe der vorliegenden Erfindung ist es, einen oder mehrere der sich aus dem Stand der Technik ergebenen Nachteile zumindest teilweise zu überwinden.

Im Speziellen basiert die Erfindung auf dem Ziel, einen Adapter für ein Mischsystem zur Bereitstellung eines Knochenzementteigs zur Verfügung zu stellen, welcher eine einfache, schnelle, anwendungssichere und kostengünstige Verbindung des Mischsystems mit einer elektrischen Antriebsvorrichtung erlaubt, mittels welcher ein Anmischen der Ausgangskomponenten des Knochenzementteigs erleichtert werden kann.

Der Adapter soll insbesondere ein möglichst einfaches und schnelles Verbinden einer elektrischen Antriebsvorrichtung mit einem Mischstab des Mischsystems erlauben, mit welchem die Ausgangskomponenten in dem Mischsystem angemischt werden können. Vorzugsweise soll der Adapter sowohl mit dem Mischsystem als auch mit der elektrischen Antriebsvorrichtung reversibel verbindbar sein.

Eine weitere Aufgabe der Erfindung besteht in der Bereitstellung eines Kits zur Bereitstellung eines Knochenzementteigs umfassend einen derartigen Adapter und ein Mischsystem, in dem die Ausgangskomponenten des Knochenzementteigs mittels eines Mischstabs vermischt werden können.

Es ist eine weitere Aufgabe der Erfindung ein Verfahren bereitzustellen, mit dem Knochenzement aus zwei Ausgangskomponenten bereitgestellt werden kann, mittels dem mindestens ein Teil der bereits beschriebenen Aufgaben zumindest zum Teil gelöst wird.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.

Eine erste Ausführungsform der Erfindung ist ein Adapter für ein Mischsystem zum Bereitstellen eines Knochenzementteigs aus zwei Ausgangskomponenten, umfassend
ein Befestigungselement zur reversiblen Befestigung des Adapters an einem Handgriff des Mischsystems, wobei der Handgriff eingerichtet ist, die zwei Ausgangskomponenten in einem Behälter des Mischsystems mittels eines Mischstab des Mischsystems zu mischen und so den Knochenzementteig bereitzustellen, und
ein Verbindungselement zum reversiblen Verbinden des Adapters mit einer elektrischen Antriebsvorrichtung,
wobei der Adapter eingerichtet ist, eine kraftübertragende Verbindung zwischen der elektrischen Antriebsvorrichtung und dem Handgriff herzustellen, so dass der Mischstab maschinell mit der elektrischen Antriebsvorrichtung betreibbar ist.

In einer Ausführungsform des Adapters weist das Befestigungselement eine Aufnahme zum Aufnehmen von mindestens 20 %, vorzugsweise von mindestens 40 %, am bevorzugtesten von mindestens 60 %, einer Oberfläche des Handgriffs, bezogen auf eine Gesamtoberfläche des Handgriffs, auf. Diese Ausführungsform ist eine zweite Ausführungsform der Erfindung, welche vorzugsweise von der ersten Ausführungsform der Erfindung abhängt.

In einer Ausführungsform des Adapters weist die Aufnahme eine innere Geometrie auf, welche im Wesentlichen komplementär zu einer äußeren Geometrie des Handgriffs ist, auf, um ein im Wesentlichen formschlüssige Verbindung des Handgriffs innerhalb des Adapters zu ermöglichen. Diese Ausführungsform ist eine dritte Ausführungsform der Erfindung, welche vorzugsweise von der zweiten Ausführungsform der Erfindung abhängt.

In einer Ausführungsform des Adapters umfasst der Adapter eine reversibel lösbare Verschlusskappe, welche eingerichtet ist, den Handgriff innerhalb der Aufnahme durch zumindest teilweises Abdecken der Aufnahme zu fixieren. Diese Ausführungsform ist eine vierte Ausführungsform der Erfindung, welche vorzugsweise von der zweiten bis vierten Ausführungsform der Erfindung abhängt.

In einer Ausführungsform des Adapters ist die Verschlusskappe durch Verrastung, durch Verklemmung, durch einen Splint und/oder durch einen Klettverschluss reversibel lösbar an der Aufnahme befestigbar, um die Aufnahme zumindest teilweise abzudecken. Diese Ausführungsform ist eine fünfte Ausführungsform der Erfindung, welche vorzugsweise von der vierten Ausführungsform der Erfindung abhängt.

In einer Ausführungsform des Adapters ist das Verbindungselement an einem dem Befestigungselement axial entgegengesetzten Ende des Adapters angeordnet. Diese Ausführungsform ist eine sechste Ausführungsform der Erfindung, welche vorzugsweise von einer der vorherigen Ausführungsformen der Erfindung abhängt.

In einer Ausführungsform des Adapters weist das Verbindungselement einen Stab mit einem in einem radialen Querschnitt runden, viereckigen oder, bevorzugt, sechseckigen Querschnitt auf. Diese Ausführungsform ist eine siebte Ausführungsform der Erfindung, welche vorzugsweise von einer der vorherigen Ausführungsformen der Erfindung abhängt.

In einer Ausführungsform des Adapters ist der Adapter derart eingerichtet, dass, bei einer Befestigung des Adapters am Handgriff des Mischsystems, der Mischstab des Mischsystems eine gemeinsame Längsachse mit dem Verbindungselement des Adapters aufweist. Diese Ausführungsform ist eine achte Ausführungsform der Erfindung, welche vorzugsweise von einer der vorherigen Ausführungsformen der Erfindung abhängt.

Eine neunte Ausführungsform der Erfindung ist ein Kit zum Bereitstellen eines Knochenzementteigs aus zwei Ausgangskomponenten umfassend einen Adapter nach einer der ersten bis achten Ausführungsform der Erfindung und ein Mischsystem, wobei das Mischsystem einen Behälter aufweist, in dem ein Knochenzementpulver als eine erste Ausgangskomponente lagerbar ist oder, bevorzugt, lagert, und einen axial im Behälter verschiebbaren und drehbar gelagerten Mischstab mit einem Handgriff, welcher von außerhalb des Behälters zugänglich ist, um den Mischstab im Inneren des Behälters zu drehen und so den Knochenzementteig bereitzustellen.

In einer Ausführungsform des Kits weist das Mischsystem ein Reservoir auf, in dem eine Monomerflüssigkeit als eine zweite Ausgangskomponente lagerbar oder, bevorzugt, lagert. Diese Ausführungsform ist eine zehnte Ausführungsform der Erfindung, welche vorzugsweise von der neunten Ausführungsform der Erfindung abhängt.

Eine elfte Ausführungsform der Erfindung ist ein Verfahren zum Bereitstellen eines Knochenzementteigs aus zwei Ausgangskomponenten mittels eines Kits nach einer der neunten oder zehnten Ausführungsform der Erfindung, umfassend die folgenden Verfahrensschritte:
a. Bereitstellen der zwei Ausgangskomponenten im Behälter;
b. Befestigen des Adapters mit dem Befestigungselement am Handgriff;
c. Verbinden des Adapters mit dem Verbindungselement an einer elektrischen Antriebsvorrichtung,
d. Mischen der Ausgangskomponenten im Behälter mittels des Mischstabs durch Betätigen der elektrischen Antriebsvorrichtung, wobei durch das Mischen der Knochenzementteig bereitgestellt wird.

Eine Ausführungsform des Verfahrens umfasst einen Verfahrensschritt e., wobei der Verfahrensschritt e. ein Lösen des Adapters vom Handgriff des Mischstabs umfasst. Diese Ausführungsform ist eine zwölfte Ausführungsform der Erfindung, welche vorzugsweise von der elften Ausführungsform der Erfindung abhängt.

Eine Ausführungsform des Verfahrens umfasst einen Verfahrensschritt f., wobei der Verfahrensschritt f. ein Austragen des Knochenzementteigs aus dem Mischsystem umfasst. Diese Ausführungsform ist eine dreizehnte Ausführungsform der Erfindung, welche vorzugsweise von der elften oder zwölften Ausführungsform der Erfindung abhängt.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen senkrechte Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B.

Die Begriffe "proximal" und "distal" dienen lediglich der Bezeichnung der räumlich gegenüberliegenden Enden der Vorrichtung oder anderer Struktureinheiten der Vorrichtung und erlauben keinen Rückschluss auf die Orientierung in Bezug zu einem menschlichen Körper, beispielsweise eines Anwenders der Vorrichtung. "Distal zu ..." und "proximal zu..." oder ähnliche Formulierungen drücken entsprechend lediglich die räumliche Anordnung zweier Struktureinheiten der Vorrichtung zueinander aus.

### Ausführliche Beschreibung

Ein erster Gegenstand der Erfindung betrifft einen Adapter für ein Mischsystem zum Bereitstellen eines Knochenzementteigs aus zwei Ausgangskomponenten, umfassend ein Befestigungselement zur reversiblen Befestigung des Adapters an einem Handgriff des Mischsystems, wobei der Handgriff eingerichtet ist, die zwei Ausgangskomponenten in einem Behälter des Mischsystems mittels eines Mischstab des Mischsystems zu mischen und so den Knochenzementteig bereitzustellen, und
ein Verbindungselement zum reversiblen Verbinden des Adapters mit einer elektrischen Antriebsvorrichtung,
wobei der Adapter eingerichtet ist, eine kraftübertragende Verbindung zwischen der elektrischen Antriebsvorrichtung und dem Handgriff herzustellen, so dass der Mischstab maschinell mit der elektrischen Antriebsvorrichtung betreibbar ist.

Der Adapter dient einem temporären, reversiblen und kraftübertragenden Verbinden eines Mischsystems zum Bereitstellen eines Knochenzementteigs aus zwei Ausgangskomponenten mit einer elektrischen Antriebsvorrichtung, wie beispielsweise einer Art elektrischer Bohrmaschine, so dass die elektrische Antriebsvorrichtung über den Adapter eine Drehbewegung auf einen Mischstab des Mischsystems übertragen kann. Der auf diese Weise drehende Mischstab dient einem Anmischen der Ausgangskomponenten im Mischsystem, insbesondere in einem Behälter des Mischsystems, unter Bereitstellen des Knochenzementteigs.

Der Adapter gibt somit einem Anwender des Mischsystem die Möglichkeit, dieses sowohl händisch, also unter Einsatz der Körperkraft zum Anmischen des Knochenzementteigs, als auch unter Zuhilfenahme einer elektrischen Antriebsvorrichtung zu betreiben. Der Adapter erhöht somit auf einfache, günstige und schnelle Weise die Flexibilität des Anwenders des Mischsystems.

Der Adapter ist eingerichtet, reversibel an einem Handgriff eines Mischsystems mit einem Befestigungselement befestigt zu werden, wobei der Handgriff wiederum direkt oder indirekt mit einem Mischstab verbunden ist. Eine Drehung des Handgriffs führt somit zu einer Drehung des Mischstabs, welcher die Ausgangskomponenten im Mischsystem zu einem Knochenzementteig anmischen kann.

Ein Handgriff eines Mischsystems erleichtert die Bedienung eines Mischstabs des Mischsystems für den Anwender, da dieser eingerichtet ist, von einer Hand des Anwenders umschlossen zu werden und so ein Drehen des Mischstabs zu erleichtern. Ein Handgriff ist ein Bauelement, welches sicher von einer Hand gegriffen werden kann, um so den Mischstab zu drehen und vorzugsweise auch axial im Mischsystem zu verschieben. Vorzugsweise ist der Handgriff ergonomisch geformt, so dass dieser leicht und sicher von einer Hand umschlossen werden kann.

Zur temporären, reversiblen Verbindung des Adapters mit einer elektrischen Antriebsvorrichtung, wie beispielsweise einer Art Bohrmaschine, weist der Adapter ein Verbindungselement auf.

Der Adapter weist somit zwei Elemente, das Befestigungselement und das Verbindungselement, um ein Mischsystem mit einer elektrischen Antriebsvorrichtung reversibel so zu verbinden, dass eine Drehbewegung der elektrischen Antriebsvorrichtung sich über den Adapter auf einen Mischstab des Mischsystems, insbesondere direkt und unmittelbar, überträgt.

Das Befestigungselement kann unterschiedlich ausgeformt sein, um reversibel an einem Handgriff eines Mischsystems befestigt zu werden.

Eine Ausführungsform des Adapters ist dadurch gekennzeichnet, dass das Befestigungselement eine Aufnahme zum Aufnehmen von mindestens 20 %, vorzugsweise von mindestens 40 %, am bevorzugtesten von mindestens 60 %, einer Oberfläche des Handgriffs, bezogen auf eine Gesamtoberfläche des Handgriffs, aufweist.

Eine Aufnahme ist ein strukturierte und/oder geformte Vertiefung, welche geeignet ist, den Handgriff in sich aufzunehmen und zumindest teilweise zu umschließen. Der Handgriff ist somit in die Aufnahme einschiebbar.

Vorzugsweise kann der gesamte Handgriff in der Aufnahme des Befestigungselement aufgenommen werden.

Die Aufnahme umschließt somit zumindest einen Teil, vorzugsweise mindestens 20 %, vorzugsweise mindestens 40 %, am bevorzugtesten mindestens 60 %, einer Oberfläche des Handgriffs, bezogen auf eine Gesamtoberfläche des Handgriffs. Die Aufnahme imitiert somit eine Hand eines Anwenders des Mischsystems, was für eine sichere Befestigung des Adapters am Mischsystem sorgt.

Eine Ausführungsform des Adapters ist dadurch gekennzeichnet, dass die Aufnahme eine innere Geometrie aufweist, welche im Wesentlichen komplementär zu einer äußeren Geometrie des Handgriffs des Mischsystems ist, um eine im Wesentlichen formschlüssige Verbindung des Handgriffs innerhalb des Adapters zu ermöglichen.

Dadurch wird eine möglichst direkte und kontrollierbare Drehung des Mischstabs mittels einer elektrischen Antriebsvorrichtung über den Adapter erreicht.

Der Adapter kann unterschiedlich ausgeformt sein, einen Mischstab sicher und reversibel in der Aufnahme zu befestigen.

Eine Ausführungsform des Adapters ist dadurch gekennzeichnet, dass dieser eine reversibel lösbare Verschlusskappe umfasst, welche eingerichtet ist, den Handgriff innerhalb der Aufnahme durch zumindest teilweises Abdecken der Aufnahme zu fixieren.

Bei geöffneter Verschlusskappe kann der Handgriff in die Aufnahme eingeführt werden und daraufhin die Verschlusskappe geschlossen werden, was den Handgriff in der Aufnahme fixiert. Die Verschlusskappe deckt im geschlossenen Zustand eine Öffnung der Aufnahme, durch welche der Handgriff in die Aufnahme eingeführt werden kann, derart ab, so dass dieser nicht mehr durch die Öffnung aus der Aufnahme entfernbar ist, ohne die Verschlusskappe geöffnet zu haben. Die Verschlusskappe ist somit eine einfache Möglichkeit, den Handgriff mechanisch in der Aufnahme zu fixieren.

Vorzugsweise sind die Aufnahme und die Verschlusskappe zweiteilig ausgeformt. Weiter bevorzugt ist der Adapter zweiteilig ausgebildet, wobei das Befestigungselement und die Aufnahme einstückig ausgebildet sind und das erste Teil des zweiteiligen Adapters ausbilden und die Verschlusskappe das zweite Teil des zweiteiligen Adapters ausbildet.

Die Verschlusskappe kann unterschiedlich ausgeformt sein, um an der Aufnahme derart reversibel befestigbar zu sein, dass die Aufnahme zumindest teilweise abgedeckt und damit der Handgriff in der Aufnahme fixiert werden kann.

Eine Ausführungsform des Adapters ist dadurch gekennzeichnet, dass die Verschlusskappe durch Verrastung, durch Verklemmung, durch einen Splint und/oder durch einen Klettverschluss reversibel lösbar an der Aufnahme befestigbar ist, um die Aufnahme, insbesondere die Öffnung der Aufnahme, zumindest teilweise abzudecken. Diese Arten an unterschiedlichen Befestigungsmöglichkeiten erlauben eine einfache und insbesondere schnelle Befestigung des Adapters am Handgriff des Mischsystems, was insbesondere im Zuge von Operationen vorteilhaft ist.

Eine Ausführungsform des Adapters ist dadurch gekennzeichnet, dass das Verbindungselement an einem dem Befestigungselement axial entgegengesetztem Ende des Adapters angeordnet ist. Vorzugsweise sind das Verbindungselement und das Befestigungselement auf einer gemeinsamen Achse, insbesondere einer Längsachse des Adapters, angeordnet. Dies erlaubt eine möglichst effiziente Kraftübertragung des Adapters von einer elektrischen Antriebsvorrichtung zum Mischsystem. Weiterhin wird so die Verwendung des Adapters erleichtert.

Das Verbindungselement kann unterschiedlich ausgestaltet sein, um mit einer elektrischen Antriebsvorrichtung reversibel verbindbar zu sein.

Vorzugsweise ist der Adapter eingerichtet, einen Handgriff eines Mischsystems reversibel mit einer elektrischen Antriebsvorrichtung zu verbinden, welche ein, vorzugsweise mit einer Drehspindel gekoppeltes, Spannfutter, vorzugsweise ein Drei-Backen-Spannfutter, aufweist. In einem derartigen System kann eine Drehung, vorzugsweise über die Drehspindel ausgeführte Drehung, über das Spannfutter und den Adapter auf das Mischsystem übertragen werden soll.

Eine Ausführungsform des Adapters ist dadurch gekennzeichnet, dass das Verbindungselement einen Stab mit einem in einem radialen Querschnitt rund, viereckigen oder, bevorzugt, sechseckigen, Querschnitt aufweist. Ein derartiger Stab lässt sich schnell, einfach und sicher in ein Spannfutter einer elektrischen Antriebsvorrichtung klemmen, so dass eine Drehbewegung einer Drehspindel über das Spannfutter und den geklemmten Adapter und letzten Endes auf einen Mischstab zum Anmischen eines Knochenzementteigs ermöglicht ist.

Eine Ausführungsform des Adapters ist dadurch gekennzeichnet, dass der Adapter derart eingerichtet ist, dass bei einer Befestigung des Adapters am Handgriff eines Mischsystems, der Mischstab des Mischsystems eine gemeinsame Achse, insbesondere Längsachse mit dem Verbindungselement des Adapters aufweist. Der Adapter fungiert in dieser Ausführungsform als eine Art Verlängerung des Mischstabs, wobei einem Anwender anstelle des Handgriffs des Mischsystems das Befestigungsmittel zur Verfügung steht, den Mischstab zu drehen. Weiterhin erlaubt diese Ausgestaltung ein effizientes und leichtes Drehen des Mischstabs über eine elektrische Antriebsvorrichtung.

Ein weiterer Gegenstand der Erfindung betrifft ein Kit zum Bereitstellen eines Knochenzementteigs aus zwei Ausgangskomponenten. Das Kit umfasst einen Adapter nach einer der vorgenannten Ausführungsformen und ein Mischsystem.

Das Mischsystem weist einen Behälter auf, in dem ein Knochenzementpulver als eine erste Ausgangskomponente lagerbar ist oder, bevorzugt, lagert, und einen reversibel axial im Behälter verschiebbaren und drehbar gelagert, insbesondere reversibel um die eigene Längsachse drehbar gelagerten, Mischstab mit einem Handgriff. Der Handgriff befindet sich außerhalb des Behälters und ist eingerichtet, den Mischstab im Inneren des Behälters zu drehen, und vorzugsweise auch innerhalb des Behälters axial zu verschieben, und so den Knochenzementteig bereitzustellen.

Die im Zusammenhang mit dem Adapter beschriebenen Merkmale eines Mischsystems sind auch für das Mischsystem des Kits zutreffend.

Eine Ausführungsform des Kits ist dadurch gekennzeichnet, dass das Mischsystem ein Reservoir aufweist, in dem eine Monomerflüssigkeit als eine zweite Ausgangskomponente lagerbar ist oder, bevorzugt, lagert. Vorzugsweise befindet sich die Monomerflüssigkeit in einem Monomerflüssigkeitsbehälter.

Ein Monomerflüssigkeitsbehälter ist ein Gefäß für die Monomerflüssigkeit, in dem diese sicher und insbesondere steril bis zu ihrer Verwendung lagerbar ist. Dem Fachmann sind unterschiedliche Monomerflüssigkeitsbehälter bekannt.

Beispielsweise kann der Monomerflüssigkeitsbehälter ein Beutel sein. Bevorzugte Beutel sind Mehrschichtverbundfolien mit einer EVOH-Sperrschicht (Ethylen-Vinylalkohol-Sperrschicht), optional umfassend eine Metallbeschichtung, insbesondere umfassend eine Aluminiumbeschichtung.

Vorzugsweise ist der Monomerflüssigkeitsbehälter eine Ampulle, vorzugsweise eine Glasampulle. Ampullen sind bevorzugt, da diese kontrollierbarer zu öffnen sind und ein vollständiges Ausfließen der Monomerflüssigkeit aus fluidleitend geöffneten Ampullen erleichtert ist.

Vorzugsweise lagert sowohl das Knochenzementpulver (im Behälter) als auch mindestens ein Monomerflüssigkeitsbehälter (im Reservoir) im Mischsystem. Dies erleichtert einem Anwender die Verwendung des Kits, insbesondere erlaubt es die Bereitstellung eines Knochenzementteigs unter sterilen Bedingungen.

Ein weiterer Gegenstand der Erfindung betrifft ein Verfahren zum Bereitstellen eines Knochenzementteigs aus zwei Ausgangskomponenten mittels eines Kits nach einer der vorhergehenden Ausführungsformen, umfassend die folgenden Verfahrensschritte:
a. Bereitstellen der zwei Ausgangskomponenten im Behälter;
b. Befestigen des Adapters mit dem Befestigungselement am Handgriff;
c. Verbinden des Adapters mit dem Verbindungselement an einer elektrischen Antriebsvorrichtung;
d. Mischen der Ausgangskomponenten im Behälter mittels des Mischstabs durch Betätigen der elektrischen Antriebsvorrichtung, wobei durch das Mischen der Knochenzementteig bereitgestellt wird.

In Verfahrensschritt a. werden die beiden Ausgangskomponenten des Knochenzementteigs im Behälter des Mischsystems des Kits zum Anmischen bereitgestellt. Vorzugsweise wird dazu die Monomerflüssigkeit als zweite Ausgangskomponente aus dem Reservoir des Mischsystems in den Behälter des Mischsystems, in welchem bereits das Knochenzementpulver als erste Ausgangskomponente lagert, gefördert.

In Verfahrensschritt b. erfolgt ein Befestigen des Adapters mit dem Befestigungselement am Handgriff des Mischsystems.

In einer bevorzugten Ausführungsform des Verfahrensschritts b. wird dazu der Handgriff in die Aufnahme des Befestigungselements, welche vorzugsweise im Wesentlichen komplementär zur äußeren Geometrie des Handgriffs ist, eingeschoben und die Aufnahme mit der reversibel lösbaren Verschlusskappe durch teilweises Abdecken der Aufnahme fixiert. Vorzugsweise werden dabei mindestens 60 % der Oberfläche des Handgriffs bezogen auf die Gesamtoberfläche des Handgriffs in die Aufnahme aufgenommen.

In Verfahrensschritt c. erfolgt ein Verbinden des Adapters mit dem Verbindungselement an einer elektrischen Antriebsvorrichtung.

In einer bevorzugten Ausführungsform des Verfahrensschrittes c. wird das Verbindungselement in Form eines in einem radialen Querschnitt sechseckigen Stab in ein Spannfutter der elektrischen Antriebsvorrichtung eingeklemmt.

Die Verfahrensschritt a., b. und c. sind in beliebiger Reihenfolge durchführbar. Vorzugsweise ist der Adapter aber zumindest mit der elektrischen Antriebsvorrichtung verbunden, bevor die Monomerflüssigkeit in das Knochenzementpulver gefördert wird.

In Verfahrensschritt d. erfolgt ein Anmischen der Ausgangskomponenten im Mischsystem unter Bereitstellung des Knochenzementteigs. Dazu wird die elektrische Antriebsvorrichtung ordnungsgemäß in Betrieb genommen, wodurch sich eine Drehbewegung der Antriebsvorrichtung über den Adapter, vorzugsweise direkt, auf den Handgriff des Mischsystems überträgt. Durch die Drehung des Handgriffs dreht sich, vorzugsweise direkt, der Mischstab des Mischsystems, was zu einem Durchmischen der Ausgangskomponenten im Mischsystem führt.

Eine Ausführungsform des Verfahrens ist dadurch gekennzeichnet, dass in einem Verfahrensschritt e. ein Lösen des Adapters vom Handgriff des Mischstabs erfolgt. Der Verfahrensschritt wird vorzugsweise nach dem Verfahrensschritt d. durchgeführt. Durch das Lösen des Adapters vom Handgriff sind die elektrische Antriebsvorrichtung und das Mischsystem wieder getrennt voneinander und ein Anwender des Kits kann das Mischsystem derart weiter benutzen, als hätte das Anmischen des Knochenzementteigs durch händisches Bedienen des Mischstabs stattgefunden. Der Adapter steht somit beispielsweise zur Verwendung mit einem weiteren Mischsystem, vorzugsweise gleicher Bauart, direkt wieder zur Verfügung.

Eine Ausführungsform des Verfahrens ist dadurch gekennzeichnet, dass in einem Verfahrensschritt f. ein Austragen des Knochenzementteigs aus dem Mischsystem erfolgt. Der Verfahrensschritt f. erfolgt nach dem Verfahrensschritt d. und vorzugsweise, falls zum Einsatz gekommen, auch nach Verfahrensschritt e.

Das Kit beziehungsweise das Mischsystem sind dadurch gekennzeichnet, dass diese einen Knochenzementteig aus zwei Ausgangskomponenten bereitstellten. Unter einem Knochenzementteig wird eine Substanz verstanden, die geeignet ist im Bereich der Medizintechnik eine stabile Verbindung zwischen künstlichen Gelenken, wie beispielsweise Hüft- und Kniegelenken, und Knochenmaterial zu erstellen. Durch Aushärtung wird aus einem Knochenzementteig ein Knochenzement. Bevorzugt handelt es sich bei diesen Knochenzementen um Polymethylmethacrylat-Knochenzemente (PMMA-Knochenzemente). PMMA-Knochenzemente kommen schon lange in medizinischen Anwendungen zum Einsatz und gehen auf Arbeiten von Sir Charnley zurück (vgl. Charnley, J. Anchorage of the femoral head prosthesis of the shaft of the femur. J. Bone Joint Surg. 1960; 42, 28-30.). PMMA-Knochenzemente können dabei aus einem Knochenzementpulver als erster Ausgangskomponente und einer Monomerflüssigkeit als zweiter Ausgangskomponente hergestellt werden. Bei geeigneter Zusammensetzung können die beiden Ausgangskomponenten getrennt voneinander lagerstabil sein. Bei Inkontaktbringen der beiden Ausgangskomponenten entsteht durch Quellung der Polymerbestandteile des Knochenzementpulvers ein plastisch verformbarer Knochenzementteig. Dabei wird eine Polymerisation des Monomers durch Radikale eingeleitet. Mit fortschreitender Polymerisation des Monomers erhöht sich die Viskosität des Knochenzementteigs, bis dieser vollständig aushärtet.

Unter einem Knochenzementpulver wird ein Pulver verstanden, welches mindestens ein partikuläres Polymethylmethacrylat und/oder ein partikuläres Polymethylmethacrylat-Copolymer umfasst. Beispiele für Copolymere sind Styren und/oder Methylacrylat. In einer Ausgestaltungsform kann das Knochenzementpulver zusätzlich ein hydrophiles Additiv umfassen, welches die Verteilung der Monomerflüssigkeit innerhalb des Knochenzementpulvers unterstützt. In einer weiteren Ausgestaltungsform kann das Knochenzementpulver zusätzlich einen Initiator, welcher die Polymerisation einleitet, umfassen. In einer weiteren Ausgestaltungsform kann das Knochenzementpulvers zusätzlich einen Röntgenopaker umfassen. In noch einer weiteren Ausgestaltungsform kann das Knochenzementpulver zusätzlich pharmazeutisch aktive Substanzen, wie beispielsweise Antibiotika, umfassen.

Bevorzugt umfasst das Knochenzementpulver als hydrophiles Additiv mindestens ein partikuläres Polymethylmethacrylat und/oder ein partikuläres Polymethylmethacrylat-Copolymer, einen Initiator und einen Röntgenopaker oder besteht aus diesen Komponenten. Weiter bevorzugt umfasst das Knochenzementpulver mindestens ein partikuläres Polymethylmethacrylat und/oder ein partikuläres Polymethylmethacrylat-Copolymer, einen Initiator, einen Röntgenopaker und ein hydrophiles Additiv oder besteht aus diesen Komponenten. Am bevorzugtesten umfasst das Knochenzementpulver mindestens ein partikuläres Polymethylmethacrylat und/oder ein partikuläres Polymethylmethacrylat-Copolymer, einen Initiator, einen Röntgenopaker, ein hydrophiles Additiv und ein Antibiotikum oder besteht aus diesen Komponenten.

Erfindungsgemäß kann die Partikelgröße des partikulären Polymethylmethacrylat und/oder des partikulären Polymethylmethacrylat-Copolymers des Knochenzementpulvers der Siebfraktion kleiner 150 µm, bevorzugt kleiner 100 µm, entsprechen.

Erfindungsgemäß kann das hydrophile Additiv partikulär und/oder faserförmig ausgestaltet sein. In einer weiteren Ausgestaltungsform kann das hydrophile Additiv schwerlöslich, bevorzugt unlöslich, in Methylmethacrylat sein. In einer weiteren Ausgestaltungsform kann das hydrophile Additiv ein Aufsaugvermögen von mindestens 0,6 g Methylmethacrylat pro Gramm hydrophiles Additiv besitzen. In einer weiteren Ausgestaltungsform kann das hydrophile Additiv eine chemische Substanz mit mindestens einer OH-Gruppe aufweisen. Dabei kann bevorzugt vorgesehen sein, dass das hydrophile Additiv kovalent gebundene OH-Gruppen an seiner Oberfläche besitzt. Beispiele für solche bevorzugten hydrophilen Additive können Additive ausgewählt aus der Gruppe umfassend Cellulose, Oxycellulose, Stärke, Titandioxid und Siliziumdioxid sein, wobei pyrogenes Siliziumdioxid besonders bevorzugt ist. In einer Ausgestaltungsform kann die Partikelgröße des hydrophilen Additivs der Siebfraktion kleiner 100 µm, bevorzugt kleiner 50 µm und am bevorzugtesten kleiner 10 µm entsprechen. Das hydrophile Additiv kann in einer Menge von 0,1 bis 2,5 Gew.-% bezogen auf das Gesamtgewicht des Knochenzementpulvers enthalten sein.

Erfindungsgemäß kann der Initiator Dibenzoylperoxid enthalten oder aus Dibenzoylperoxid bestehen.

Erfindungsgemäß versteht man unter einem Röntgenopaker eine Substanz, welche es erlaubt, den Knochenzement auf röntgendiagnostischen Aufnahmen sichtbar zu machen. Beispiele für Röntgenopaker können Bariumsulfat, Zirkondioxid und Kalziumcarbonat umfassen. Erfindungsgemäß kann die pharmazeutisch aktive Substanz ein oder mehrere Antibiotika und gegebenenfalls zugesetzte Co-Faktoren für das eine oder die mehreren Antibiotika umfassen. Bevorzugt besteht die pharmazeutisch aktive Substanz aus einem oder mehreren Antibiotika und gegebenenfalls zugesetzten Co-Faktoren für das eine oder die mehreren Antibiotika. Beispiele für Antibiotika sind unter anderem Gentamicin, Clindamycin und Vancomycin. Erfindungsgemäß kann die Monomerflüssigkeit das Monomer Methylmethacrylat umfassen oder aus Methylmethacrylat bestehen. In einer Ausgestaltungsform umfasst die Monomerflüssigkeit neben dem Monomer einen darin gelösten Aktivator, wie beispielsweise N,N-Dimethyl-p-toluidin, oder besteht aus Methylmethacrylat und N,N-Dimethyl-p-toluidin.

Die im Zusammenhang mit dem Adapter offenbarten Merkmale sind auch für das Kit, das Mischsystem sowie das Verfahren offenbart und umgekehrt.

### Figuren

Die Erfindung wird im Folgenden durch Figuren weiter beispielhaft illustriert. Die Erfindung ist nicht auf die Figuren beschränkt.

Es zeigen
- Fig. 1: eine perspektivische Seitenansicht eines beispielhaften Kits zum Bereitstellen eines Knochenzementteigs umfassend einen Adapter und ein Mischsystem sowie eine perspektivische Seitenansicht einer elektrischen Antriebsvorrichtung,
- Fig. 2: einen schematischen Längsschnitt durch die Bestandteile des Kits und die elektrische Antriebsvorrichtung aus Figur 1,
- Fig. 3: einen schematischen Längsschnitt und eine schematische Seitenansicht des Adapters aus den Figuren 1 und 2 jeweils mit einer geöffneten und einer geschlossenen Verschlusskappe,
- Fig. 4: einen schematischen Längsschnitt des Kits und der elektrischen Antriebsvorrichtung aus den vorherigen Figuren, wobei der Adapter über ein Verbindungselement mit der elektrischen Antriebsvorrichtung verbunden ist,
- Fig. 5: einen schematischen Längsschnitt des Kits und der elektrischen Antriebsvorrichtung aus den vorherigen Figuren, wobei der Adapter über das Verbindungselement mit der elektrischen Antriebsvorrichtung und über ein Befestigungselement mit einem Handgriff des Mischsystems verbunden ist,
- Fig. 6: eine perspektivische Seitenansicht des Kits und der elektrischen Antriebsvorrichtung aus den vorherigen Figuren, wobei der Adapter über das Verbindungselement mit der elektrischen Antriebsvorrichtung und über das Befestigungselement mit dem Handgriff des Mischsystems verbunden ist,
- Fig. 7: eine schematische Seitenansicht eines weiteren beispielhaften Kits zum Bereitstellen eines Knochenzementteigs umfassend einen Adapter und ein Mischsystem, und
- Fig. 8: ein Flussdiagramm eines Verfahrens zum Bereitstellen eines Knochenzementteigs.

### Beschreibung der Figuren

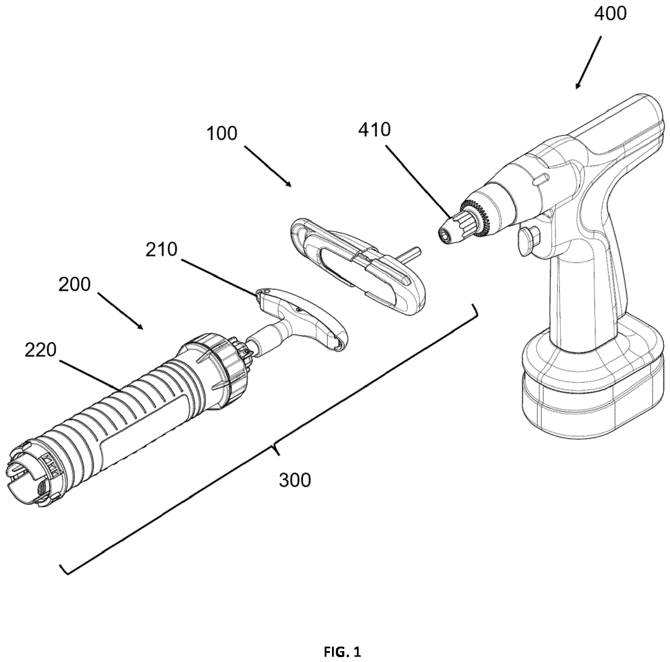

**Figur 1** zeigt eine perspektivische Seitenansicht eines beispielhaften Kits 300 zum Bereitstellen eines Knochenzementteigs aus zwei Ausgangskomponenten sowie eine perspektivische

Seitenansicht einer beispielhaften elektrischen Antriebsvorrichtung 400. Das Kit 300 umfasst einen Adapter 100 und ein Mischsystem 200 mit einem Handgriff 210. Der Adapter 100 ist eingerichtet, sowohl mit dem Handgriff 210 des Mischsystems 200 als auch mit einem Spannfutter 410 der elektrischen Antriebsvorrichtung 400 derart verbunden zu werden, dass zwischen dem Handgriff 310 und der elektrischen Antriebsvorrichtung 400 eine kraftübertragende Verbindung besteht, so dass eine Drehbewegung der elektrischen Antriebsvorrichtung 400 über den Adapter 100 auf den Handgriff 210 übertragbar ist, was ein Bereitstellen eines Knochenzementteigs in einem Behälter 220 des Mischsystems 200 erlaubt. Die Funktionsweise wird im Zusammenhang mit den folgenden Figuren näher beschrieben.

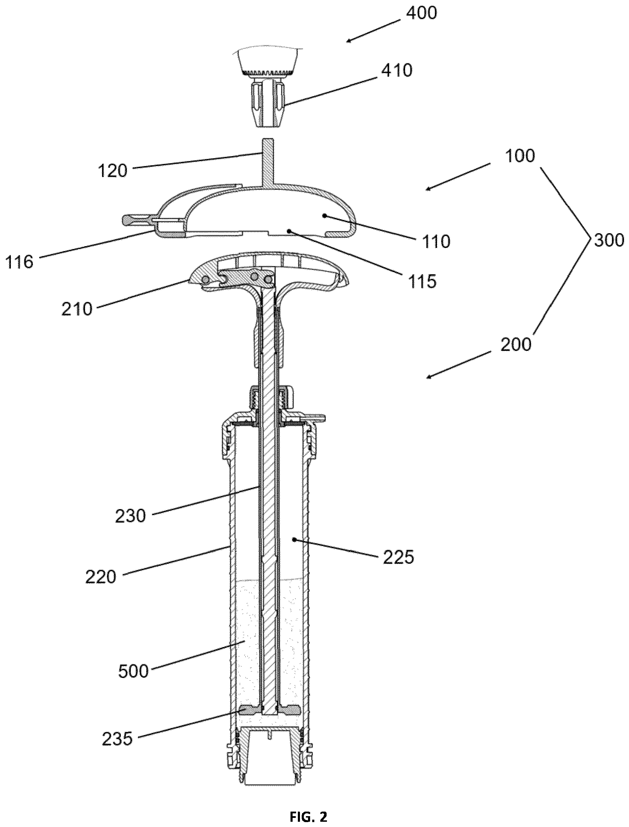

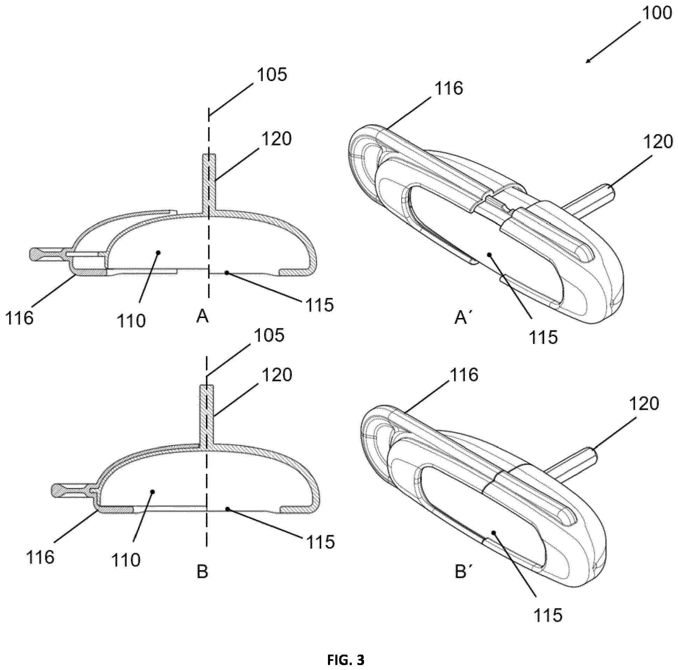

**Figur 2** zeigt das Kit 300 und einen Abschnitt der elektrischen Antriebsvorrichtung 400 aus Figur 1 in einem schematischen Längsschnitt. Der Adapter 100 weist ein Befestigungselement 110 in Form einer Aufnahme zum Aufnehmen des Handgriffs 210 des Mischsystems 200 auf. Die Aufnahme 110 ist ausgeformt, den Handgriff 210 vollständig aufzunehmen und weist eine innere Geometrie auf, welche im Wesentlichen komplementär zu einer äußeren Geometrie des Handgriffs ist. Die gezeigte Ausführungsform ermöglicht somit ein im Wesentlichen formschlüssiges Verbinden des Handgriffs 210 innerhalb des Adapters 100, insbesondere innerhalb des Befestigungselements 110 des Adapters 100. Um den Handgriff 210 im Befestigungselement 110 nach dessen Einführen zu fixieren, weist der Adapters 100 eine reversibel lösbare Verschlusskappe 116 auf, welche sich in Figur 2 in einem geöffneten Zustand befindet. Im geöffneten Zustand der Verschlusskappe 116 ist ein Ein- und Ausführen des Handgriffs 210 in das Befestigungselement 110 möglich. Zur Funktionsweise der Verschlusskappe 116 wird auf die folgenden Figuren, insbesondere Figur 3, verwiesen. An einem dem Befestigungselement 110 axial entgegengesetztem Ende weist der Adapter 100 ein Verbindungselement 120 in Form eines Stabs, insbesondere eines sechseckigen Stabs (vgl. Figur 3) auf. Das Befestigungselement 120 kann in das Spannfutter 410 der elektrischen Antriebsvorrichtung 400 eingeführt und dort fixiert werden.

In Figur 2 ist ersichtlich, dass der Handgriff 210 mit einem Mischstab 230 verbunden ist, welcher in einen Innenraum 225 des Behälters 220 des Mischsystems 200 hineinragt und dort drehbar und axial verschiebbar gelagert ist. Der Mischstab 230 weist an einem dem Handgriff 210 axial entgegengesetztem Ende ein Mischelement 235 in Form von radial abstehenden Flügeln auf, welche ein Anmischen der Ausgangskomponenten des Knochenzementteigs erleichtern.

Als erste Ausgangskomponente des Knochenzementteigs lagert im Innenraum 225 ein Knochenzementpulver 500.

**Figur 3** zeigt den Adapter 100 aus den Figuren 1 und 2 sowohl in einem schematischen Längsschnitt (3A und 3B) als auch in einer perspektivischen Seitenansicht (3A' und 3B'). Die Verschlusskappe 116 befindet sich in den Figuren 3A und 3A', wie auch bereits in Figur 2 gezeigt, im geöffneten Zustand. Im geöffneten Zustand ist die Verschlusskappe 116 radial von einer Längsachse 105 des Adapters 100, welche sich durch das Verbindungselement 120 erstreckt, nach außen verschoben. Dadurch liegt die Öffnung 115 des Befestigungselements 110 derart frei, dass der Handgriff 410 des Mischsystems 200 (vgl. bspw. Figur 2) in den Adapter 100 einbringbar ist, um dort im Befestigungselement 110 fixiert zu werden. In den Figuren 3B und 3B' befindet sich die Verschlusskappe 116 hingegen in einem geschlossenen Zustand. Die Verschlusskappe 116 ist durch ein Verschieben derselben in Richtung der Längsachse 105 vom geöffneten in den geschlossenen Zustand reversibel überführbar. Im geschlossenen Zustand kann die Verschlusskappe 116 den Handgriff 210 des Mischsystems 200 im Befestigungselement 110 fixieren, so dass eine Drehbewegung, welche auf das Verbindungselement 120 ausgeübt wird, sich über das Befestigungselement 110 auf den Handgriff 210 übertragen kann. Dazu verdeckt die Verschlusskappe 116 einen Teil der Öffnung 115 des Befestigungselements 110, was ein Ausbringen des im Befestigungselements 110 vorhandenen Handgriffs 210 aus demselben verhindert. Um die Verschlusskappe 116 sicher im geschlossenen Zustand zu sichern, ist diese reversibel am Befestigungselement 110 durch Verrastung befestigt.

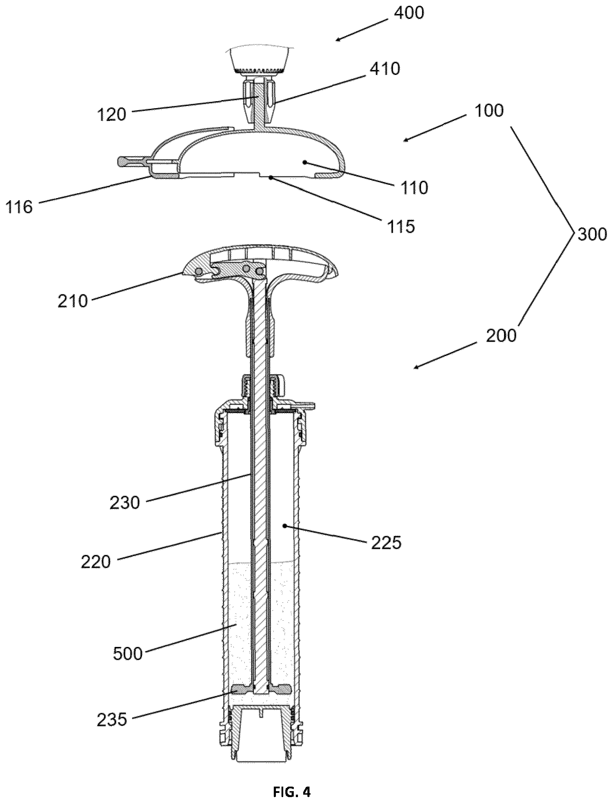

**Figur 4** zeigt das Kit 300 und die elektrische Antriebsvorrichtung aus den Figuren 1 und 2 in einem schematischen Längsschnitt, wobei das Verbindungselement 120 in das Spannfutter 410 der elektrischen Antriebsvorrichtung 400 eingeschoben und dort fixiert ist. Die Verschlusskappe 116 befindet sich, wie auch schon in Figur 2 gezeigt, im geöffneten Zustand, so dass der Handgriff 410 in das Befestigungselement 110 eingeschoben werden kann.

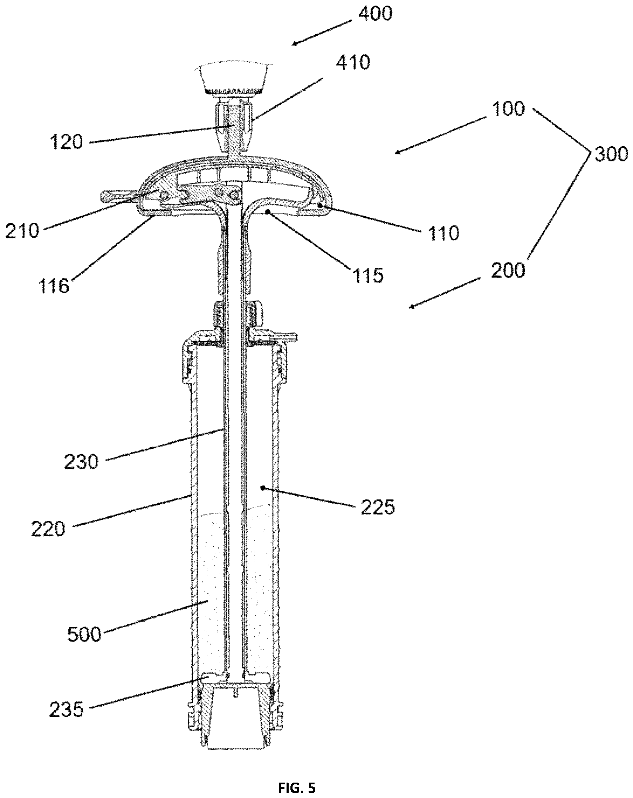

**Figur 5** zeigt das Kit 300 und die elektrische Antriebsvorrichtung aus den Figuren 1, 2 und 4 in einem schematischen Längsschnitt, wobei zusätzlich zu Figur 4 der Handgriff 210 des Mischsystems 200 im Befestigungsmittel 110 fixiert wurde. Dazu wurde die Verschlusskappe 116 in Richtung der Längsachse 105 des Adapters 100 (vgl. Figur 3) verschoben, wodurch diese im geschlossenen Zustand am Befestigungsmittel 110 verrastet und somit befestigt wurde. Der Adapter 100 erlaubt in Figur 5 somit eine kraftübertragende Verbindung zwischen der elektrischen Antriebsvorrichtung 400, insbesondere dem Spannfutter 410, und dem Handgriff 210 des Mischsystems 200. Eine durch eine ordnungsgemäße Betätigung der elektrischen Antriebsvorrichtung 400 ausgelöste Drehung übertragt sich somit direkt auf den Mischstab 230, was ein Anmischen des Knochenzementteigs unter Bereitstellen desselben erlaubt. In Figur 5 fehlt hierzu lediglich ein Bereitstellen einer Monomerflüssigkeit als zweite Ausgangskomponente im Innenraum 225 des Behälters 220.

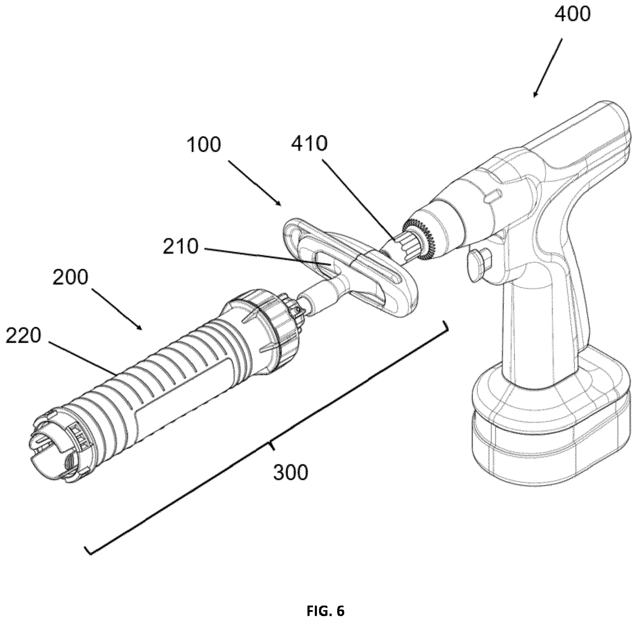

**Figur 6** zeigt Kit 300 und die elektrische Antriebsvorrichtung aus den Figuren 1, 2, 4 und 5 in einer perspektivischen Seitenansicht, wobei, wie in Figur 5 beschrieben, der Adapter 100 sowohl mit der elektrischen Antriebsvorrichtung 400 als auch mit dem Handgriff 210 verbunden ist und somit eine kraftübertragende Verbindung zwischen diesen beiden hergestellt hat. Durch eine ordnungsgemäße Betätigung der elektrischen Antriebsvorrichtung 400 können somit im Mischsystem 200, insbesondere im Innenraum 225 (vgl. bspw. Figur 2) des Behälters 220 die Ausgangskomponenten des Knochenzementteigs angemischt werden und der Knochenzementteig dadurch bereitgestellt werden.

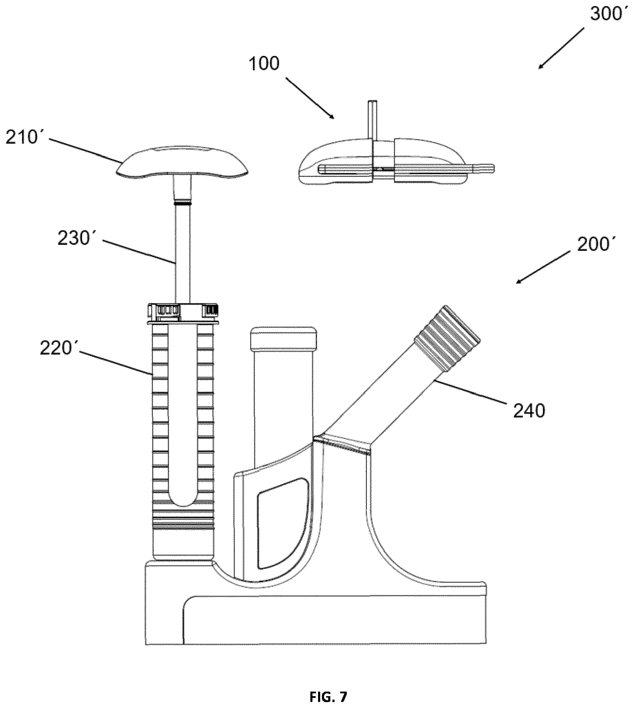

**Figur 7** zeigt ein weiteres beispielhaftes Kit 300' zum Bereitstellen eines Knochenzementteigs aus zwei Ausgangskomponenten in einer schematischen Seitenansicht. Das Kit 300' umfasst einen Adapter 100, wie er bereits in den vorherigen Figuren beschrieben wurde, und ein weiteres beispielhaftes Mischsystem 200'. Das Mischsystem 200' unterscheidet sich von dem Mischsystem 200 der vorherigen Figuren durch ein zusätzliches Reservoir 240, in dem eine mit einer Monomerflüssigkeit als zweite Ausgangskomponente gefüllte Glasampulle lagert (nicht gezeigt). Weiterhin lagert in einem Behälter 210' des Mischsystems 200' ein Knochenzementpulver als erste Ausgangskomponente (nicht gezeigt). Für den genauen Aufbau und die Funktionsweise des Mischsystems 200' wird hiermit auf die Offenbarung der Anmeldung EP3093067A1 Bezug genommen.

Bezüglich des Einsatzes des Adapters 100 mit dem Mischsystem 200', insbesondere mit dessen Handgriff 210', wird auf die vorherigen Figuren verwiesen, da das Mischsystem 200' sich an den hierfür entscheidenden Stellen nicht wesentlich vom Mischsystem 200 der vorherigen Figuren unterscheidet. Eine Verwendung des Adapters 100 mit dem Mischsystem 200' ist daher im Wesentlichen analog zu den vorbeschriebenen Figuren möglich.

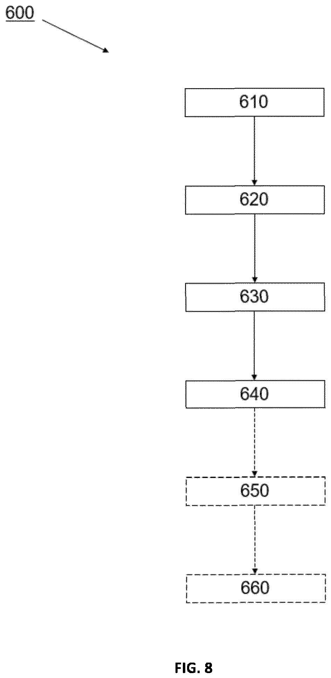

**Figur 8** zeigt ein beispielhaftes Verfahren 600 zur Bereitstellung eines Knochenzementteigs aus zwei Ausgangskomponenten mittels eines Kits 300, 300' aus den vorherigen Figuren umfassend die Verfahrensschritte 610 bis 640 sowie optional die Verfahrensschritt 650 und/oder 660, wobei die Reihenfolge der Verfahrensschritte 610 bis 630 auch in einer anderen als der gezeigten Reihenfolge stattfinden können.

In einem Verfahrensschritt 610 erfolgt ein Bereitstellen der beiden Ausgangskomponenten im Behälter 210, 210', insbesondere im Innenraum 225 des Behälters 210, 210', des Mischsystems 200, 200'. Vorzugsweise lagert das Knochenzementpulver 500 als erste Ausgangskomponente des Knochenzementteigs bereits im Behälter 210, 210' bevor die Monomerflüssigkeit als zweite Ausgangskomponente in den Behälter 210, 210' gefördert wird. Die Monomerflüssigkeit lagert vorzugsweise vor dem Fördern in den Behälter 210, 210' in einem Reservoir 240 des Mischsystems 200'.

In einem Verfahrensschritt 620 erfolgt ein Befestigen des Adapters 100 mittels des Befestigungselements 110 am Handgriff 210, 210' Mischsystems 200, 200'. Vorzugsweise wird hierzu der Handgriff 210, 210' in eine Aufnahme des Befestigungselements 110 eingebracht und dort mittels einer Verschlusskappe 116 fixiert.

In einem Verfahrensschritt 630 erfolgt ein Verbinden des Adapters 100 mittels des Verbindungselements 120, vorzugsweise in Form eines Stabs, vorzugsweise eines sechseckigen Stabs, an einer elektrischen Antriebsvorrichtung 400, vorzugsweise an einem Spannfutter 410 einer elektrischen Antriebsvorrichtung.

Durch die Verfahrensschritte 620 und 630 wird eine kraftübertragende Verbindung zwischen der elektrischen Antriebsvorrichtung 400 und dem Handgriff 210, 210' hergestellt, so dass der Mischstab 230, 230' maschinell mit der elektrischen Antriebsvorrichtung betreibbar ist.

In einem Verfahrensschritt 640 erfolgt ein Mischen der im Behälter in Verfahrensschritt 610 bereitgestellten Ausgangskomponenten des Knochenzementteigs. Dazu wird die elektrische Antriebsvorrichtung 400 ordnungsgemäß betätigt, beispielsweise durch Betätigung eines Einschaltknopfes der elektrischen Antriebsvorrichtung 400, wodurch sich eine Drehbewegung der elektrischen Antriebsvorrichtung 400 über den Adapter 100 und den Handgriff 210, 210'auf den Mischstab 230, 230' des Mischsystems 200, 200' übertragt. Der so von der elektrischen Antriebsvorrichtung 400 angetriebene Mischstab 230, 230' durchmischt die bereitgestellten Ausgangskomponenten unter Bereitstellung des Knochenzementteigs.

In einem optionalen Verfahrensschritt 650 kann der Adapter 100 wieder vom Handgriff 210, 210' des Mischsystems 200, 200' gelöst werden. Dies erlaubt ein weiteres händisches Betätigen des Handgriff 210, 210' und/oder ein weiteres Verwenden des Adapters 100 mit einem weiteren Mischsystem 200, 200'.

In einem optionalen Verfahrensschritt 660 erfolgt ein Austragen des im Zuge des Verfahrens 600 bereitgestellten Knochenzementteigs aus dem Mischsystem 200, 200'. Vorzugsweise wird dazu zuerst Verfahrensschritt 650 durchgeführt.

### Bezugszeichen

- 100: Adapter
- 110: Befestigungselement
- 115: Öffnung
- 116: Verschlusskappe
- 120: Verbindungselement
- 200, 200': Mischsystem
- 210,210': Handgriff
- 220,220': Behälter
- 225: Innenraum
- 230, 230': Mischstab
- 235: Mischelement
- 240: Reservoir
- 300, 300': Kit
- 400: elektrische Antriebsvorrichtung
- 410: Spannfutter
- 500: Knochenzementpulver
- 600: Verfahren
- 610: Bereitstellen
- 620: Befestigen
- 630: Verbinden
- 640: Mischen
- 650: Lösen
- 660: Austragen

## Patentansprüche

1. Adapter (100) für ein Mischsystem (200, 200') zum Bereitstellen eines Knochenzementteigs aus zwei Ausgangskomponenten, umfassend
ein Befestigungselement (110) zur reversiblen Befestigung des Adapters (100) an einem Handgriff (210, 210') des Mischsystems (200, 200'), wobei der Handgriff (210, 210') eingerichtet ist, die zwei Ausgangskomponenten in einem Behälter (220, 220') des Mischsystems (200, 200') mittels eines Mischstab (230, 230') des Mischsystems (200, 200') zu mischen und so den Knochenzementteig bereitzustellen, und
ein Verbindungselement (120) zum reversiblen Verbinden des Adapters (100) mit einer elektrischen Antriebsvorrichtung (400),
wobei der Adapter (100) eingerichtet ist, eine kraftübertragende Verbindung zwischen der elektrischen Antriebsvorrichtung (400) und dem Handgriff (210, 210') herzustellen, so dass der Mischstab (230, 230') maschinell mit der elektrischen Antriebsvorrichtung (400) betreibbar ist.

2. Adapter (100) nach Anspruch 1, wobei das Befestigungselement (110) eine Aufnahme zum Aufnehmen von mindestens 20 %, vorzugsweise mindestens 40 %, am bevorzugtesten mindestens 60 %, einer Oberfläche des Handgriffs (210, 210'), bezogen auf eine Gesamtoberfläche des Handgriffs (210, 210'), aufweist.

3. Adapter (100) nach Anspruch 2, wobei die Aufnahme eine innere Geometrie aufweist, welche im Wesentlichen komplementär zu einer äußeren Geometrie des Handgriffs (210, 210') ist, um eine im Wesentlichen formschlüssige Verbindung des Handgriffs (210, 210') innerhalb des Adapters (100) zu ermöglichen.

4. Adapter (100) nach einem der Ansprüche 2 bis 4, umfassend eine reversibel lösbare Verschlusskappe (116), welche eingerichtet ist, den Handgriff (210, 210') innerhalb der Aufnahme durch zumindest teilweises Abdecken der Aufnahme zu fixieren.

5. Adapter (100) nach Anspruch 4, wobei die Verschlusskappe (116) durch Verrastung, durch Verklemmung, durch einen Splint und/oder durch einen Klettverschluss reversibel lösbar an der Aufnahme befestigbar ist, um die Aufnahme zumindest teilweise abzudecken.

6. Adapter (100) nach einem der vorherigen Ansprüche, wobei das Verbindungselement (120) an einem dem Befestigungselement (110) axial entgegengesetztem Ende des Adapters (100) angeordnet ist.

7. Adapter (100) nach einem der vorherigen Ansprüche, wobei das Verbindungselement (120) einen Stab mit einem in einem radialen Querschnitt runden, viereckigen oder, bevorzugt, sechseckigen Querschnitt aufweist.

8. Adapter (100) nach einem der vorhergehenden Ansprüche, wobei der Adapter (100) derart eingerichtet ist, dass, bei einer Befestigung des Adapters (100) am Handgriff (210, 210') des Mischsystems (200, 200'), der Mischstab (210, 210') des Mischsystems (200, 200') eine gemeinsame Längsachse mit dem Verbindungselement (120) des Adapters (100) aufweist.

9. Kit (300, 300') zum Bereitstellen eines Knochenzementteigs aus zwei
Ausgangskomponenten, umfassend einen Adapter (100) nach einem der Ansprüche 1 bis 8 und ein Mischsystem (200, 200'), wobei das Mischsystem (200, 200') einen Behälter (220, 220') aufweist, in dem ein Knochenzementpulver (500) als eine erste Ausgangskomponente lagert oder lagerbar ist, und
einen axial im Behälter (220, 220') verschiebbaren und drehbar gelagerten Mischstab (230, 230') mit einem Handgriff (210, 210'), welcher von außerhalb des Behälters (220, 220') zugänglich ist, um den Mischstab (230, 230') im Inneren des Behälters (220, 220') zu drehen und so den Knochenzementteig bereitzustellen.

10. Kit (300') nach Anspruch 9, wobei das Mischsystem (200') ein Reservoir (240) aufweist, in dem eine Monomerflüssigkeit als eine zweite Ausgangskomponente lagert oder lagerbar ist.

11. Verfahren (600) zum Bereitstellen eines Knochenzementteigs aus zwei Ausgangskomponenten mittels eines Kits (300, 300') nach einem der Ansprüche 9 oder 10, umfassend die folgenden Verfahrensschritte:
a. Bereitstellen (610) der zwei Ausgangskomponenten im Behälter (220, 220');
b. Befestigen (620) des Adapters (100) mit dem Befestigungselement (110) am Handgriff (210, 210');
c. Verbinden (630) des Adapters (100) mit dem Verbindungselement (120) an einer elektrischen Antriebsvorrichtung (400);
d. Mischen (640) der Ausgangskomponenten im Behälter (220, 220') mittels des Mischstabs (230, 230') durch Betätigen der elektrischen Antriebsvorrichtung (400), wobei durch das Mischen (640) der Knochenzementteig bereitgestellt wird.

12. Verfahren (600) nach Anspruch 11, umfassend einen Verfahrensschritt e., wobei der Verfahrensschritt e. ein Lösen (650) des Adapters (100) vom Handgriff (210, 210') des Mischstabs (230, 230') umfasst.

13. Verfahren (600) nach Anspruch 11 oder 12, umfassend eine Verfahrensschritt f., wobei der Verfahrensschritt f. ein Austragen (660) des Knochenzementteigs aus dem Mischsystem (200, 200') umfasst.
